# EUROPEAN PATENT SPECIFICATION

(11) **EP 1 265 648 B1**
(45) Date of publication and mention of the grant of the patent: **12.03.2008**
(21) Application number: 00916914.5
(22) Date of filing: 09.03.2000
(51) Int. Cl.: A61L 15/32, A61F 13/00

(54) **NOVEL collagen-BASED MATERIAL WITH IMPROVED PROPERTIES FOR USE IN HUMAN AND VETERINARY MEDICINE AND THE METHOD OF MANUFACTURING SUCH**
NEUES MATERIAL AUF KollagenBASIS MIT VERBESSERTEN EIGENSCHAFTEN ZUR VERWENDUNG IN DER HUMAN- UND VETERINÄRMEDIZIN UND EIN HERSTELLUNGSVERFAHREN
NOUVEAU MATERIAU A BASE DE collagène, QUI PRESENTE DES PROPRIETES AMELIOREES ET EST DESTINE A UN USAGE EN MEDECINE HUMAINE ET VETERINAIRE, ET SON PROCEDE DE PRODUCTION

(43) Date of publication of application: 18.12.2002
(73) Proprietor: Syntacoll AG, 9100 Herisau (CH)
(72) Inventor: RUSZCZAK, Zbigniew, D-12557 Berlin (DE); MEHRL, Robert, D-84085 Langweid (DE); JECKLE, Johann, D-93339 Riedenburg (DE); STOLTZ, Michael, D-85540 Haar (DE)
(74) Representative: Böhm, Brigitte
(86) International application number: PCT/EP2000/002056
(87) International publication number: WO 2001/066159

(56) References cited:
- DE-A- 2 843 963
- DE-A- 4 027 887
- US-A- 4 578 067
- US-A- 5 567 806
- DATABASE WPI Section Ch, Week 200005 Derwent Publications Ltd., London, GB; Class A32, AN 1991-314990 XP002901384 & JP 02 992590 B (NIPPON VALQUA IND LTD), 20 December 1999 (1999-12-20)

## Description

The present invention relates to novel collagen-based material with improved mechanical, physical, functional and handling properties for use in human and veterinary medicine in both in vivo and in vitro condition and to methods for the manufacture of such material.

The use of various different xenogenous, allogenic or autologous collagen-based materials in human and veterinary medicine in both experimental (i.e. ex vivo) and in vivo condition is known. Such a collagen material may be used for example as hemostatic agent, as substitute of missing tissue, as skin equivalent, as material for tissue augmentation or as a carrier for biologically active substances or drugs.

Purified allogenic or xenogenous collagen is almost fully biocompatible with human collagenous and connective tissue and may be incorporated into and/or subsequently remodeled to a host tissue without foreign body reaction and immunologic rejection.

If used as a hemostatic agent, collagen-based material must have both biological and mechanical features promoting hemostasis, i.e., intact collagen fibers and an optimal porosity.

If used as tissue substitute (equivalent), the collagen-based material must have optimal matrix properties promoting formation of granulation tissue, angiogenesis, vascularization and epithelization.

If used as a carrier of biologically active substances, the collagen-based material must have features allowing an optimal release of incorporated agent(s) as well as good matrix properties.

In all cases, however, the handling of the collagen-based material, its mechanical stability, flexibility and, if necessary, the ability to be sutured or sealed are important factors characterizing a good, ready-to-use and user-friendly material.

The most popular commercially available collagen-based materials are sponges, membranes or injectable solutions of various fibril contents and viscosity.

For hemostasis, tissue substitution and as a carrier for biologically active substances both lyophilized collagen-based sponges and/or air-dried membranes are the most popular.

All these known materials, however, are not stable enough to be sutured, rolled, screwed or sticked, or to be used in areas of mechanical tension.

To improve the mechanical properties of such collagen materials, different additional crosslinking procedures have been developed. The most popular are: chemical crosslinking (i.e. with aldehydes) or physical crosslinking (i.e. dehydro-thermal treatment).

The aldehyde-based crosslinking may negatively influence the biocompatibility of collagen and may lead to some residues of aldehydes (or its derivatives) in the final product.

The dehydro-thermal treatment, which is used mostly for collagen sponges, has also its natural limitation and does not lead to materials with sufficiently improved properties.

To overcome these problems different alternative manufacturing processes have been described.

The US-Patent 4,655,980 describes the possible manufacturing of a collagen membrane based on a soluble collagen gel suspension. The membrane may be obtained by applying pressure to the gel, or by disrupting the gel and separating the resulting precipitate for casting. Depending on the dimension and shape of the casting mold, either a membrane or solid can be obtained. In fact, the manufacturing of such membrane is based on a commercially available soluble, injectable, atelocollagen product of Collagen Aesthetics, Paolo Alto, CA, USA.

The US-Patent 5,219,576 describes a collagen implant material useful as wound healing matrices and delivery system for bioactive agents. Beside manufacturing traditional collagen sponges based on casting and drying of a soluble collagen gel suspension, the patent describes the manufacturing of multilayer material by casting and freezing the individual layers and then lyophilizing the entire composite at once. A possibility of additional crosslinking by both aldehyde and dehydro-thermal processing of the final product is also discussed.

The US-Patent 4,522,753 describes, for example, a method for preserving porosity and improving stability of collagen sponges by both aldehyde and dehydro-thermal treatment. The negative pressure (vacuum) used may vary from about 1 mtorr up to slight vacuum just below atmospheric pressure.

The US-Patent 4,578,067 describes a hemostatic-adhesive collagen dressing in form of dry-laid, non-woven, self-supporting webs of collagen fibers. The manufacturing of such material is based on a Rando-feeder and Rando-webber techniques. The collagen fibers from the Rando-feeder are introduced into the air stream of the Rando-webber and form a fiber mass of uniform density. Such mass may then be processed by pressing or embossing or by calendering at a temperature ranging from room temperature to 95°C.

The US-Patent 5,206,028 describes a collagen membrane having improved physical and biological properties. Such membrane does not swell appreciably upon being wetted and maintains its density. The manufacturing of such translucent, collagen Type-I based material is based on compression of collagen sponges in a roller press with a calibrate aperture followed by aldehyde cross-linking. For additional mechanical stabilization, the cross-linked membrane may be re-wetted, re-lyophilized and pressed again under standard conditions.

The US-Patent 4,948,540 describes a mechanically stable, comfortable collagen wound dressing sheet material fabricated by lyophilizing a collagen composition (soluble and insoluble collagen parts in range of 1:20 to 10: 1) and compressing the porous pad at a pressure between about 15,000 and 30,000 p.s.i. The material may be also cross-linked by dehydro-thermal treatment to improve mechanical stability.

At present, all of the methods for manufacturing of collagen-based material with improved mechanical, physical and biological properties (as described above) are not in use for industrial manufacturing of collagen-based material.

There is a need, however, both in human and veterinary medicine, to use and introduce an industrially manufactured, ready-to-use, customer friendly collagen-based material which will be fully biocompatible, mechanically stabile, flexible, easy-to-handle, and which can be sutured or/and sealed, rolled or/and screwed, cut or/and meshed.

Moreover, it would be of advantage, if such material could contain biologically active substances or/and agents, e.g., to promote healing or to protect from (or cure) infections.

The most important, however, the new product has to be easy to manufacture and not expensive.

An object of the present invention, therefore, was to create a novel collagen-based product with improved mechanical and/or physical and/or bio-physiological properties which can be easily manufactured by industrial methods.

Moreover, a further object of the present invention was to create a material with novel mechanical and/or physical and/or bio-physiological properties which allows the successful use of the material in areas and/or indications in which such properties are necessary.

These objects are solved according to the invention by providing a collagen-based material with improved mechanical, physical, functional and handling properties for use in human and veterinary medicine in both in vivo and in vitro condition and wherein
the material is a collagen of animal, or/and human or/and recombinant or/and transgenic origin optionally containing additionally
(a) biologically active substances such as hemostatic agents, growth factors, cytokines, hormones, drugs (such as antibiotics, antiinflammatory agents) etc.,
   and/or
(b) biologically important and tissue-compatible inorganic or/and organic substances or/and their derivatives which can improve the mechanical, functional, biological, and handling properties of the material,
obtainable by simultaneously treating a basic collagen sponge material made from a dispersion or/and suspension of 0.5 - 5 weight% of collagen at 50°C-200°C and 0.5 to 1000 kg/cm² to form a membrane-like material and, if applicable, adding the additional biological substance(s) of a) and/or b) to the collagen material prior or subsequent to the heat and pressure treatment.

According to the invention, defined mechanical pressure and defined heat are applied in a way which protects the fibrils (native and/or renaturate) of the collagen or the other natural polymers from degradation or/and denaturation or/and melting and which save the natural biological properties of collagen (i.e. hemostatic properties or matrix properties). Moreover, the method by which the collagen-based material of the invention is prepared, allows to create and manufacture collagen-based material with improved mechanical properties like stability, dry and wet tension, suturing abilities, improved flexibility, with excellent hemostatic properties, improved wetting abilities, improved absorption of water or/and other physiologic fluids and which can be rolled or/and screwed in both dry and wet condition, and which can be cut or meshed without loosing shape and basic properties.

Moreover, the present invention allows the manufacturing of collagen-based products which may contain biologically active agents and/or inorganic or/and organic substances of the kind as described above and which can additionally serve as a carrier for living cells, tissue sealant, etc.

The present invention opens new possibilities for the use of collagen-based materials in those areas of the human or animal body or in those in vivo and ex vivo indications in which the use of such biological material was previously not possible or applicable due to, i.e., insufficient mechanical properties.

The manufacturing steps used for the preparation of the material and described in the present invention can be easily incorporated into a routine manufacturing process and allow to save time and costs if compared to other currently used methods (see above).

The use of defined mechanical pressure for industrial manufacture of collagen membrane-like products based on freeze-dried collagen sponges containing active substances, i.e., antibiotics (i.e. gentamicin) is known per se (i.e. EP 0069260, issued 09/25/1985, owned by Syntacoll AG, Herisau, Switzerland).

The influence of a moderate heat, especially if used together with a negative pressure (vacuum), for induction of additional cross-linking sites in collagen sponges has been described previously as dehydro-thermal-treatment (see above).

The present invention now combines heat and positive pressure (mechanical pressure) for the treatment of the basic materials according to the invention, which has never been proposed in the state of the art, but leads to products with highly unexpected, superior properties as described above.

The collagen used for manufacture of the improved collagen-based material of the invention may be either of animal origin (xenogenous) or human (autologous or allogenic) origin or may be obtained from genetically manipulated organisms (recombinant techniques or transgenic organisms) which can produce recombinant or transgenic proteins, or can be obtained by other techniques of genetic engineering, equivalent or similar.

The collagen material used for manufacturing of the improved collagen-based material may consist of various known collagen types (preferentially of at least one or/and or all of the following: Typ-I, -II, III, IV, VII, IX alone or in a mixture). The most important collagen in the human and animal body is the Typ-I collagen. This material can be easily obtained for example from animal tissue (skin, tendons, etc.,) by industrial methods according to state-of-the-art, GMP-conformed techniques.

Both enzymatically treated or not enzymatically treated collagen can be used for manufacture. If treated with proteolytic enzymes, non-helical parts of the collagen molecule will be separated from the triple-helical collagen chain(s) (atelocollagen).

The basis material for manufacturing an improved collagen-based material is a collagen sponge. The basis for such material may be collagen dispersion and/or suspension (i.e. in water or other non-organic solvent) of 0.5 to 5.0 weight% of dry collagen. A sponge of defined porosity may be obtained by freeze-drying. The collagen sponge may be manufactured using various state-of-the-art techniques.

To improve mechanical properties, handling and to achieve new features of the collagen-based material collagen sponges with various different, but defined water (solvent) contents will be treated simultaneously with defined continuos heat and defined continuos mechanical pressure for a defined period of time.

Preferably, according to the present invention, the water or solvent content ranges from 2 % to 40 % of weight.

The temperature used lies within the range of from 50 °C to 200 °C.

The pressure used lies in the range of from 0,5 kg/cm² to 1000 kg/cm².

The time period for the heat and pressure treatment preferably lies within the range of from 0.1 second to 1 hour.

As a result of such treatment according to the invention, a collagen-based sponge will be pressed to a membrane-like structure. This product has excellent hemostatic properties, excellent and improved absorbing capacity, excellent and improved mechanical properties (rolling, screwing, suturing, etc.,).

Moreover, such a product shows much better handling properties than other known collagen based products such as freeze-dried sponges or air-dried membranes.

Additionally, the new properties of such material allow its use in those anatomic sites or medical indications, in which the use of traditional collagen-based sponges or membrane was hithero not possible or not practicable.

Moreover, the new properties of the material of the invention allow the use of sutures or other methods of mechanical fixation in situ, which was not possible in the case of traditional collagen-based materials.

The final product according to the present invention may be packed using any suitable packaging and end-sterilized by, i.e., ethylene oxide vapors, gamma radiation, electron beam radiation or any other sterilization procedure suitable for such material.

Another subject of the present invention is a process for the manufacture of a collagen based material having improved mechanical and/or physical, and/or functional and/or handling properties for use in human and veterinary medicine in both in vivo and in vitro condition and described above in more detail, by simultaneously treating a basic collagen material with defined heat and defined pressure.

The manufacturing of the novel collagen-based material is easy and can be incorporated into standard industrial production lines. The necessary equipment is commercially available.

For the process according to the invention, as basic collagen material a sponge made from a dispersion or/and suspension with a collagen content of 0.5 to 5 weight% is used. The temperature used for the heat treatment lies within the range of from 50 to 200 °C and the pressure used for the pressure treatment lies within the range of from 0,5 to 1000 kg/cm². Preferably the treatment time lies within 0.1 second to 1 hour.

The following examples are intended to further illustrate the invention.

### Example 1

### Manufacturing of a novel collagen-based membrane-like material

A freeze-dried collagen sponge, like i.e. Collatamp® (manufacturer: SYNTACOLL AG, Herisau, Switzerland) with a thickness of 5 mm and a collagen content of 5,6 mg/cm³ is conditioned in a moisture chamber to a water content of 14 % of weight.

After conditioning, defined mechanical pressure and defined heat are applied simultaneously to the sponge using an appropriate press.

The pressure of 5 kg/cm² is applied continuously to both sites of the sponge for a time period of 10 seconds; the temperature of pre-heated press surfaces is 80°C and remains constant during pressing.

After such thermal pressing procedure (ThermPress™), the resulting paper-like collagen-membrane has a thickness of 0,1 mm.

Physical properties of the new created product are markedly improved, if compared to a standard collagen sponge: (1) the product is easy to handle and may be rolled or screwed without breaking, (2) the swelling time of the product is dramatically reduced, (3) the absorption of fluids increases, (3) the wet product (after fluid absorption and/or swelling) remains very flexible, (4) the wet product has much better elasticity and excellent wet tensile strength.

The novel features of the product make it very interesting for use in general surgery, vascular surgery, neurology and neurosurgery, orthopedics and orthopedic surgery, cardiosurgery, gynecologic surgery, ophthalmology, laryngology, and in all other medical and veterinary disciplines including wound healing and burns.

Moreover, the novel product may have benefit if used as a tissue substitute or as a matrix for cell growth especially in tissue engineering and creation of artificial organs.

### Example 2

### Manufacturing of a novel collagen-sheet with improved swelling properties

A freeze-dried collagen-based sponge (collagen content 30 mg/cm³) with a thickness of 5 mm is conditioned in a moisture chamber to have a water content of 14% of weight.

After conditioning, pressure and heat are applied simultaneously to the sponge.

The pressure of 5 kg/cm² is applied from both sides of the sponge for a time of 10 seconds. The temperature of pre-heated press surfaces is 80°C and remains constant during pressing.

After such a thermal pressing procedure (ThermPress™), the resulting collagen sheet is 0,6 mm thick and had the appearance of a strong paper or is leather-like. If compared to standard freeze-dried collagen sponge, such collagen sheet has dramatically improved swelling properties.

Moreover, the swelling time is markedly reduced and fluid binding capacity is increased.

Such collagen sheet can swell to 30 times it's weight in maximal time of 10 seconds. Additionally, hemostatic properties are markedly increased. In swollen condition the collagen sheet is mechanical stable and has a high wet tensile strength. Both the dry and the wet sheet is very easy to handle, can be cut to any desired or suitable form. Due to its high stiffness and high elasticity, it is more easy to apply it to different locations in the body.

The novel features of this product make it very interesting especially for use in hemostasis (properties much better than all standard hemostatic agents as e.g. standard collagen sponge, gelatin sponge, regenerating cellulose, cotton gaze, etc.). Moreover, the product may have benefits in general surgery, vascular surgery, neurology and neurosurgery, orthopedics and orthopedic surgery, cardiosurgery, gynecologic surgery, ophthalmology, laryngology, and in all other medical and veterinary disciplines including wound healing and burns.

Moreover, the novel product may have benefit if used as a tissue substitute or as a matrix for cell growth, especially in tissue engineering and creation of artificial organs.

Additionally the novel product may be use as a carrier for biologically active substances as i.e. growth factors, cytokines, hormones, drugs, etc., which can be added ex tempore and/or incorporated to the product by absorption.

As the basic collagen sponge used for thermal press procedure can contain biologically active substances as i.e. growth factors, cytokines, hormones, drugs, etc., novel product created by the method described by the present invention can also contain such substances. However, in such cases the manufacturing condition have to be adapted to save the biologic activity of the particular additive.

## Claims

1. Collagen-based material with improved mechanical, physical, functional and handling properties for use in human and veterinary medicine in both in vivo and in vitro condition and wherein
the material is a collagen of animal, or/and human or/and recombinant or/and transgenic origin optionally containing additionally
(a) biologically active substances such as hemostatic agents, growth factors, cytokines, hormones, drugs (such as antibiotics, antiinflammatory agents) etc.,
and/or
(b) biologically important and tissue-compatible inorganic or/and organic substances or/and their derivatives which can improve the mechanical, functional, biological, and handling properties of the material,
obtainable by simultaneously treating a basic collagen sponge material made from a dispersion or/and suspension of 0.5 - 5 weight% of collagen at 50°C-200°C and 0.5 to 1000 kg/cm² to form a membrane-like material and, if applicable, adding the additional biological substance(s) of a) and/or b) to the collagen material prior or subsequent to the heat and pressure treatment.

2. Collagen-based material according to claim 1, wherein the time of simultaneous application of the continuous heat and pressure lies within 0.1 second and 1 hour.

3. Process for the manufacture of a collagen-based material according to claims 1 or 2, **characterized by** simultaneously treating a basic collagen sponge material made from a dispersion or/and suspension of 0.5 to 5 weight% of collagen at 50°C-200°C and 0.5 to 1000 kg/cm².

4. Process according to claim 3, wherein the time of simultaneous application of continuous heat and pressure lies within 0.1 seconds and 1 hour.

## Patentansprüche

1. Material auf Kollagenbasis mit verbesserten mechanischen, physikalischen, funktionellen und Handhabungseigenschaften zur Verwendung in der Human- und Veterinärmedizin unter In vivo- und In vitro-Bedingung und wobei das Material ein Kollagen tierischen oder/und menschlichen oder/und rekombinanten oder/und transgenen Ursprungs ist, wobei es optional zusätzlich enthält
(a) biologisch aktive Substanzen wie hämostatische Mittel, Wachstumsfaktoren, Zytokine, Hormone, Medikamente (wie Antibiotika, antiinflammatorische Mittel etc.) und/oder
(b) biologisch wichtige und gewebekompatible anorganische oder/und organische Substanzen oder/und deren Derivate, die die mechanischen, funktionellen, biologischen und Handhabungseigenschaften des Materials verbessern können,
erhältlich durch gleichzeitiges Behandeln eines Kollagenschwamm-Ausgangsmaterials, das aus einer Dispersion oder/und Suspension mit 0,5-5 Gewichts% Kollagen gemacht ist, bei 50°C-200°C und 0,5 bis 1000 kg/cm², um ein membranähnliches Material zu formen und, gegebenenfalls, Zugeben der zusätzlichen biologischen Substanz(en) aus (a) und/oder (b) zum Kollagenmaterial vor oder nach der Hitze- und Druckbehandlung.

2. Material auf Kollagenbasis gemäß Anspruch 1, wobei die Zeit der gleichzeitigen Anwendung der kontinuierlichen Hitze und des kontinuierlichen Drucks zwischen 0,1 Sekunde und 1 Stunde beträgt.

3. Verfahren zur Herstellung eines Materials auf Kollagenbasis gemäß den Ansprüchen 1 oder 2, **dadurch gekennzeichnet, dass** ein Kollagenschwamm-Ausgangsmaterial, das aus einer Dispersion oder/und Suspension von 0,5 bis 5 Gewichts-% Kollagen gemacht ist, gleichzeitig bei 50°C-200°C und 0,5 bis 1000 kg/cm² behandelt wird.

4. Verfahren nach Anspruch 3, wobei die Zeit der gleichzeitigen Anwendung von kontinuierlicher Hitze und Druck zwischen 0,1 Sekunde und 1 Stunde beträgt.

## Revendications

1. Matériau à base de collagène avec des propriétés mécaniques, physiques, fonctionnelles et manipulatrices améliorées pour l'utilisation en médecine humaine et vétérinaire à la fois en conditions in vivo et in vitro et dans lequel
le matériau est un collagène d'origine animale et/ou humaine et/ou recombiné et/ou transgénique contenant optionnellement de plus
(a) des substances biologiquement actives, telles que des agents hémostatiques, des facteurs de croissance, des cytokines, des hormones, des médicaments (tels que des antibiotiques, des anti-inflammatoires), etc.,
et/ou
(b) des substances organiques ou/et inorganiques, biologiquement importantes et compatibles avec les tissus ou/et leurs dérivés qui peuvent améliorer les propriétés mécaniques, fonctionnelles, biologiques et de manipulation du matériau,
pouvant être obtenu par traitement simultané à 50°C-200°C et 0,5 à 1000 kg/cm² d'un matériau d'éponge collagène de base réalisé à partir d'une dispersion ou/et d'une suspension de 0,5 à 5 % en poids de collagène, pour former un matériau de type membrane et, au besoin, ajouter la ou les substances biologiques supplémentaires de a) et/ou b) au matériau collagène avant ou après le traitement thermique et de compression.

2. Matériau à base de collagène selon la revendication 1, dans lequel le temps d'application simultanée de la chaleur et de la compression continues se situe dans un espace de temps de 0,1 seconde à 1 heure..

3. Procédé pour la fabrication d'un matériau à base de collagène selon les revendications 1 ou 2, **caractérisé par** le traitement simultané d'un matériau d'éponge collagène de base réalisé à partir d'une dispersion ou/et d'une suspension de 0,5 à 5 % en poids de collagène à 50°C-200°C et 0,5 à 1000 kg/cm².

4. Procédé selon la revendication 3, dans lequel le temps d'application simultanée de la compression et de la chaleur en continu se situe dans un espace de temps de 0,1 seconde à 1 heure.
